**Europäisches Patentamt**

(19) **European Patent Office**

**Office européen des brevets**

(11) Numéro de publication: **0 035 919**

**A2**

(12) # DEMANDE DE BREVET EUROPEEN

(21) Numéro de dépôt: **81400170.7**

(22) Date de dépôt: **04.02.81**

(51) Int. Cl.³: **A 61 K 7/06**

(30) Priorité: **05.03.80 FR 8004886**

(43) Date de publication de la demande:
**16.09.81 Bulletin 81/37**

(84) Etats contractants désignés:
**BE CH DE GB IT LI NL**

(71) Demandeur: **Frajdenrajch, Sophie**
**50 Boulevard du Général Leclerc**
**F-92200 Neilly S/Seine(FR)**

(72) Inventeur: **Frajdenrajch, Sophie**
**50 Boulevard du Général Leclerc**
**F-92200 Neilly S/Seine(FR)**

(74) Mandataire: **Cuer, André**
**CABINET CUER 30, rue de Léningrad**
**F-75008 Paris(FR)**

(54) **Composition pour le traitement des cheveux.**

(57) L'invention a trait à une nouvelle composition capillaire apte à assurer un véritable traitement scientifique du cheveu.

La composition se présente sous la forme d'une solution alcoolique renfermant essentiellement comme ingrédients actifs : alcoolat de térébenthine composé , teinture d'arnica, extrait de ginseng, sel de chondroïtine ou mucopolysaccharides de cartilage; et, de préférence : teinture de quinquina, extrait parfumé (eau de Cologne, de citron..), D. panthénol, vitamine H et vitamine E; éventuellement : extrait de cresson et cystéine.

Les résultats obtenus sont spectaculaires quant au stoppage de la chute et à la repousse des cheveux, les solutions étant en outre non toxiques et non irritantes.

La présente invention a trait au domaine des produits capillaires et concerne particulièrement une nouvelle composition, sous forme de solution alcoolique, apte à assurer un véritable traitement scientifique du cheveu notamment par contrôle de la séborrhée.

La chute des cheveux, due notamment /à l'âge, à la maladie ou /à un état séborrhéique du cuir chevelu, souvent aggravé par les fréquentes décolorations et teintures, constitue une véritable maladie dont la progression est malheureusement constatée chaque année chez certains peuples et dans de nombreux milieux. Le cuir chevelu dont l'avenir sera mauvais se couvre de pellicules (ou pityriasis capitis) qui, d'abord sèches au jeune âge ou à l'adolescence, deviennent graisseuses chez l'adulte et entraînent la chute progressive des cheveux. Une telle alopécie, souvent provoquée par ailleurs du fait de diverses affections, est d'autant plus désagréable que, souvent limitée à des aires bien déterminées comme le sommet de la tête ou les régions temporales, elle devient parfois calvitie totale et exige l'adoption de perruques ou moyens similaires.

Outre l'hygiène préventive, divers traitements ont été préconisés pour lutter notamment contre la séborrhée tels que des frictions à cadences régulières à l'aide de lotions alcoolisées à base de produits tels que du formol, de la quinine, des oestrogènes de synthèse, de la pilocarpine ou encore des compositions renfermant du sélénium. Ces traitements sont parfois efficaces mais ils présentent pour la plupart de sérieux inconvénients. Par exemple, les produits contenant du sélénium ou encore du Jaborandi (à base de pilocarpine) sont toxiques et donc inutilisables pour un traitement prolongé ; d'ailleurs les composés actifs correspondants sont classés respectivement dans les groupes A et C de la classification officielle des produits dangereux en pharmacie. D'autre part les hormones femelles, souvent administrées, entraînent chez l'homme une féminisation progressive et sont donc anti-virulisants.

L'invention a pour but la mise au point d'une solution de traitement qui permet d'obvier aux divers inconvénients précités. Elle propose à cet effet une composition destinée à stimuler la circulation sanguine sur le cuir chevelu et dont l'effet est double à savoir de stopper la chute des cheveux en un temps relativement court et de provoquer rapidement la pousse de nouveaux cheveux sur les zones atteintes d'alopécie.

La nouvelle composition selon l'invention se présente sous la forme d'une solution alcoolique contenant essentiellement les ingrédients actifs suivants : alcoolat de térébenthine composé, teinture d'Arnica, extrait de ginseng et un sel de chondroïtine ou des mucopolysaccharides de cartilage.

Comme on le sait, l'alcoolat de térébentine composé est un mélange complexe d'un grand nombre, environ une quinzaine de substances médicinales. Il a été trouvé, de façon surprenante, qu'il possédait d'excellentes propriétés stimulantes dans le traitement du cuir chevelu, lorsqu'il est combiné aux ingrédients précités. L'extrait du ginseng semble avoir une activité similaire à la folliculine sans cependant présenter d'effets secondaires féminisants. En pratique, cet extrait peut-être utilisé sous diverses formes telles que poudres, teintures, etc. Selon une réalisation avantageuse, on peut employer un extrait glycolique titré à 0,5 à 2 % de saponines ou encore un extrait hydroalcoolique titré à 5-10 %. Quant à la chondroïtine, constituée, comme on le sait, d'extraits de collagène, elle peut être mise en oeuvre sous diverses formes, comme par exemple du sulfate. L'excipient alcoolique est généralement sous forme d'alcool éthylique utilisé à 90 degrés en mélange avec de l'eau distillée pour avoir un degré alcoolique final dans la composition compris entre 65 et 75 degrés.

Conformément à une réalisation particulièrement avantageuse, la composition selon l'invention renferme, outre les constituants précités, les produits choisis dans le groupe : teinture de quinquina, extrait parfumé comme par exemple eau de Cologne à 80° ou essence de lavande ou citron, ainsi que du panthénol tel que le D-panthénol dont on connait déjà l'action bénéfique sur le cheveu.

Il a en outre été trouvé que l'addition de la solution d'une petite quantité de vitamine H (biotine) et de vitamine E (tocophénol) procurait un effet synergique sur les constituants précités et rendait encore plus spectaculaires les résultats.

Selon, enfin, une autre variante, la composition précitée peut par ailleurs contenir de la cystine (ou cystéine) et un extrait ou une teinture de cresson.

En pratique, les proportions des divers ingrédients dans les compositions de l'invention peuvent varier entre d'assez larges limites. Généralement, ces dernières sont constituées par les fourchettes suivantes : 0,5 à 4 % pour l'alcoolat de térébenthine composé; 0,5 à 2 % pour la teinture de quinquina ; 0,25 à 1 % de teinture d'arnica ; 0,1 à 0,5 % d'essence de lavande ou citron ou encore 1 à 2 % d'eau de Cologne à 80° ; 0,1 à 1 % de D-panthénol ; 2 à 12 % d'extrait de ginseng (par exemple sous forme d'extrait glycolique titré à 1 % de saponines); 0,1 à 1,5 % de sel de chondroitine ; 0,1 à 1,5 de vitamine H et 0,3 à 3 % de vitamine E ; 1 à 2 % de cresson ; 0,1 à 0,5 % de cystine; la quantité d'excipient aloolique jusqu'à 100 % étant constituée par de l'éthanol 90° dilué par de l'eau minéralisée jusqu'à un degré alcoolique de 65 à 75°.

A titre d'exemple non limitatif de réalisation on peut citer la composition suivante, selon la variante où la totalité des ingrédients précités est mise en oeuvre :

| | | |
|---|---|---|
| teinture de quinquina | : | 1 % |
| teinture d'arnica | | 1 % |
| alcoolat de térébenthine composé | : | 1 % |
| Eau de Cologne à 80 % | : | 1 % |
| D. panthénol | : | 0,3 % |
| extrait glycolique de ginseng titré à 1 % de saponines | : | 5 % |
| Sulfate de chondroitine : | : | 0,5 % |
| biotine | : | 0,6 % |
| tocophérol | : | 0,8 % |
| éthanol à 90° et eau déminéralisée pour avoir solution à 72° | : q.s. pour 100 % | |

les pourcentages étant indiqués en poids.

Les compositions capillaires selon l'invention ont fait l'objet de nombreux essais et expertises cliniques qui ont mis en évidence les propriétés et caractéristiques suivantes :

. stimulation de la circulation sanguine sur le cuir chevelu et parfait contrôle de la séborrhée ;

. stoppage de la chute des cheveux après un traitement variant entre 2 et 3 semaines

. repousse de nouveaux cheveux généralement dès la troisième ou quatrième semaine après le début des applications

. absence totale de toxicité et aucune irritation constatée, quelle que soit la durée d'administration (essais effectués après plusieurs mois de traitement.

. aucun pouvoir allergène : le test de MAGNUSSON s'est avéré négatif

. excellente tolérance : les tests de toxicité itérative et de tolérance oculaire se sont révélés négatifs.

En pratique, l'application locale des solutions capillaires selon l'invention peut se faire soit par frottement au tampon soit, de préférence par vaporisation sur les racines des cheveux et/ou sur le cuir chevelu sec en insistant sur les zones dégarnies. Un massage vigoureux est ensuite conseillé pour faciliter la pénétration de la solution dans l'épiderme. Il est conseillé d'effectuer le traitement matin et soir pendant une période d'environ trois semaines, délai habituel à l'issue duquel les effets sont nettement visibles. Pour parfaire les résultats, les applications sont de préférence poursuivies pendant deux à trois mois ; la durée de traitement pouvant de toute façon être prolongée étant donné la totale innocuité de la solution.

REVENDICATIONS
-----------------------------

1. Composition capillaire destinée notamment à stopper la chute et faire repousser les cheveux constituée par une solution alcoolique caractérisée en ce qu'elle renferme essentiellement comme constituants actifs la combinaison d'ingrédients suivants : alcoolat de térébenthine composé, teinture d'Arnica, extrait de ginseng et un sel de chondroitine.

2. Composition selon la revendication 1, caractérisée en ce que le sel de chondroitine est remplacé par des mucopolysaccharides de cartilage.

3. Composition selon l'une quelconque des revendications 1 ou 2, caractérisée en ce qu'elle renferme en outre de la teinture de quinquina, un produit parfumé choisi dans le groupe : eau de Cologne, essence de lavande ou de citron, du D-panthénol, l'excipient alcoolique étant constitué par de l'alcool éthylique de degré 65 à 75.

4. Composition selon l'une quelconque des revendications 1 ou 2, caractérisée en ce que le ginseng est mis en oeuvre sous forme d'extrait glycolique titré à 1 % de saponines ou d'extrait hydroalcoolique à 5-10 % de saponines et en ce que le sel de chondroitine est constitué par du sulfate.

5. Composition selon l'une quelconque des revendications 1 à 4, caractérisée en ce qu'elle renferme en outre de la biotine (ou vitamine H) et du tocophénol (ou vitamine E).

6. Composition selon l'une quelconque des revendications 1 à 5, caractérisée en ce qu'elle contient également de la cystine (ou cystéine) et un extrait ou teinture de cresson.

7. Composition selon la revendication 6, caractérisée en ce qu'elle se présente sous la forme d'une solution vraie contenant :

| | |
|---|---|
| Alcoolat de térébenthine | : 0,5 à 4 % |
| Teinture de Quinquina | : 0,5 à 2 % |
| Teinture d'Arnica | : 0,25 à 1 % |
| Eau de Cologne (ou essence de lavande ou citron) | : 1 à 2 % |
| D-panthénol | : 0,1 à 1 % |
| Extrait glycolique ou hydroalcoolique de ginseng titré à 1-10 % de saponines | : 2 à 12 % |
| Sulfate de chondroitine | : 0,1 à 1,5 % |
| Vitamine H | : 0,1 à 1,5 % |

| vitamine E | : 0,3 à 3 % |
| cystine | : 0,1 à 0,5 % |
| extrait de cresson | : 1 à 2 % |
| Ethanol à 90° et eau déminéralisée | : q.s. pour 100 % |

de façon à avoir un degré alcoolique de 65 à 75.

les pourcentages étant indiqués en poids.

         8. Composition selon la revendication 7, caractérisée en ce qu'elle renferme (pourcentages en poids)

| Alcoolat de térébenthine composé | : 1 % |
| Teinture de quinquina | : 1 % |
| Teinture d'arnica | : 1 % |
| Eau de Cologne à 80° | : 1 % |
| (ou équivalent) | |
| D-panthénol | : 0,3 % |
| Extrait glycolique de | |
| ginseng à 1 % de saponines | : 5 % |
| Sulfate de chondroitine : | : 0,5 % |
| Vitamine H | : 0,6 % |
| Vitamine E | : 0,8 % |
| Eventuellement : | |
| Extrait de Cresson | : 2 % |
| cystéine | : 0,2 % |
| éthanol étendu à 72° | : q.s. pour 100 % |